# EUROPEAN PATENT APPLICATION

(11) **EP 4 154 899 A1**
(43) Date of publication of application: **29.03.2023**
(21) Application number: 21809722.8
(22) Date of filing: 19.05.2021
(51) Int. Cl.: A61K 38/00, A61P 29/00

(54) **DRUG WITH PROLONGED ANALGESIC ACTION**

(30) Priority: 20.05.2020 RU 2020117918
(71) Applicant: Federalnoe Gosudarstvennoe Budzhetnoje Uchrezhdenie Nauki Tikhookeansky Institut Bioorganicheskoi Khimii Im. G.B. Elyakova, Vladivostok, 690022 (RU)
(72) Inventor: LEYCHENKO, Elena Vladimirovna, Vladivostok, 690022 (RU); SINTSOVA, Oksana Vladimirovna, Partizansk, 692860 (RU); GLADKYKH, Irina Nikolaevna, Vladivostok, 690066 (RU); KLIMOVICH, Anna Anatolievna, Strugovka, 692574 (RU); MONASTYRNAYA, Margarita Mikhailovna, Vladivostok, 690105 (RU); DIACHENKO, Igor Aleksandrovich, Puschino, 142290 (RU); MURASHEV, Arkady Nikolaevich, Puschino, 142290 (RU); MOSHAROVA, Irina Vladimirovna, Moscow, 105082 (RU); KOZLOV, Sergey Aleksandrovich, Moscow, 121069 (RU); KOZLOVSKAYA, Emma Pavlovna, Vladivostok, 690022 (RU)
(74) Representative: Rimini, Rebecca
(86) International application number: PCT/RU2021/050133
(87) International publication number: WO 2021/235983

(57) **Abstract**

The invention relates to biochemistry, more specifically to the use of biologically active peptides having an inhibitory action on pain receptors, and concerns methods for using the HCRG21 peptide from a sea anemone *Heteractis crispa* as an analgesic dmg which has a lengthy analgesic effect on account of inhibiting the functional activity of the TRPV1 ion channel. Said peptide can be used as a medicinal drug independently or in a mixture with other active components in order to reduce pain syndromes of various etiology, including acute and chronic pain syndromes, to relieve inflammation, and also to achieve a moderate hypothermal effect. The invention makes it possible to produce an effective long-acting analgesic preparation having a specific action on a cellular target and which can be delivered into the body either non-invasively or parenterally.

## Description

### Field of the invention

The invention relates to biochemistry, more specifically to the use of biologically active peptides as analgesics that can be useful in medicine and veterinary medicine for reducing pain syndromes including acute and chronic as well as in research studies for developing methods of discovery of new promising drugs, or as tools for investigating molecular organization and mechanisms of operation of ion channels, receptors and proteolytic enzymes.

### Background of the invention

Pain is an important physiological phenomenon which is for the most part accompanied by the development of pathologies. There is an acute and chronic pain. Unlike a chronic pain, an acute pain is as a rule a short-term one, has an easily identified cause and usually yields well to treatment. A chronic pain can be a nociceptive (induced by a tissue injury or inflammation) or neuropathic one. A neuropathic pain results from a pathological neuron excitation in the peripheral or central nervous system and is maintained by disturbed somatosensorial processes. The therapy of pain conditions conventionally includes a combined use of non-steroidal anti-inflammatory drugs and other non-opioid analgesics such as acetylsalicylic acid and derivatives thereof, acetaminophen, ibuprofen, fenoprofen, diflunisal and naproxen; and/or opioid analgesics including morphine, hydromorphone, methadone, levorphanol, fentanyl, oxycodone and oxymorphone. The use of opioids is related to numerous side effects including physical dependence and drug addiction. It is proved that a fast growth of the use of prescription opioid drugs in the USA correlates well with the increase of opioid overdose mortality rate and the increase in illicit drug trafficking. Opioid abuse and dependency are growing from year to year and this is leading the world community in general to a severe crisis that has crippling effects on the public health and economy. Unfortunately, at present there are no viable alternatives to replace opioid analgesics in circumstances of acute postoperative pains and oncology diseases.

The development of breakthrough analgesics able to control molecular mechanisms of pain generation is impossible without the understanding of a target specificity of the study drug. It is determined that receptors and ion channels of nociceptive peripheral terminals and axons are key players in pain signaling processes (Basbaum A.I. et al. Cellular and molecular mechanisms of pain // Cell 2009, 139, 267-284). They specifically include nonselective cation channels of the TRP (Transient Receptor Potential) family involved in various physiological processes in living organisms, such as photoreception, pheromone perception, taste perception, pain, mechanoreception, blood pressure regulation etc. For mammals it was shown that the malfunction of the channels is directly related to the development of a different pathological conditions (Mickle A.D. et al. Nociceptive TRP Channels: Sensory Detectors and Transducers in Multiple Pain Pathologies // Pharmaceuticals 2016, 9, e72).

Some types of TRP channels are used by animals as thermoreceptors to control the body temperature and to determine the environment temperature, what is particularly topical to protect from a temperature harmful for vital activity of an organism and to ensure the survival of an organism. Among so called "thermo" TRP channels, the most characterized is a TRPV1 (transient receptor potential vanilloid type 1) nonselective cation channel favoring Ca²⁺ (Szallasi A. The vanilloid receptor TRPY1: 10 years from channel cloning to antagonist proof-of-concept // Nat. Rev. Drug Discov. 2007, 6, 357-372). It is a polymodal ion channel activated by different chemical substances, such as capsaicin from chili pepper, resiniferatoxin from gum euphorbia, camphor from camphor tree, peptide toxins, endogenous lipids (anandamide, N-arachidonoyl dopamine, leukotriene B4) known as "endovanilloids", and is responsive to the intercellular acidification (pH<5.9). However the most important function thereof in mammals consists in the activation by a harmful temperature (> 42°C) (Starowicz K. et al. Biochemistry and pharmacology of endovanilloids // Pharmacol. Ther. 2007, 114:13-33; Cao E., et al. TRPV1 channels are intrinsically heat sensitive and negatively regulated by phosphoinositide lipids // Neuron 2013, 77, 667-679). The TRPV1 is also indirectly activated and sensitized by a wide range of other inflammation mediators like histamine, bradikinin, prostaglandins and ATP as well as by NGF, which among others increases the TRPV1 expression in sensory neurons (RU 2621708, RU 2648445, RU 2541127, RU 2506077, RU 2379282, EP 2352726, US 2017266139).

The TRPV1 is expressed in cells of the central or peripheral nervous system and is involved in many significant physiological processes such as thermoregulation, lipogenesis, bladder function, cardiac function, brain neurogenesis.

Numerous studies showed that the channel is involved in the regulation of a number of pathological conditions, such as a gastroesophageal reflux disease, fecal/urinary incontinence, allergic contact dermatitis, respiratory hypersensitivity, cardiovascular disorders, diabetic and peripheral neuropathy and cancer pains (Jara-Oseguera A. et al. TRPV1: On the road to pain relief // Curr. Mol. Pharmacol. 2008, 1, 255-269; Mickle A.D. et al. Nociceptive TRP Channels: Sensory Detectors and Transducers in Multiple Pain Pathologies // Pharmaceuticals 2016,9, pii.E72).

TRPV1 channel agonists capsaicin and resiniferatoxin (RTX) are used in medical practice for a long time as analgesics (US 5178879, US 566378, US 6239180, US 4313958, US 4599342, US 5188837), they are also used for activation or ablation of TRPV1 expressing cells, what gives an idea on a role of these specific nociceptors in somatic or visceral pains (Cianchetti C. Capsaicin jelly against migraine pain // Int. J. Clin. Pract. 2010, 64,457-459; Myzsik G. Capsaicin as new orally applicable gastroprotective and therapeutic drug alone or in combination with nonsteroidal anti-inflammatory drugs in healthy human subjects and in patients // Prog. Drug Res. 2014, 68, 209-258). In the patent literature the practical use of these agonists is mentioned in the documents of Internationally renowned companies: Glenmark Pharmaceuticals S.A. (WO2007042906, WO2008110863, WO2008059339, WO2009095726, WO2009010824, WO2009081219, WO2009081222, WO2008010061, WO2009090548, WO2009034433), Gruenenthal GmbH (WO2007045462, WO2008125295, WO2008125296, WO2008125337, WO2008125342, WO2008046647), PharmEste s.r.l. (WO 2008075150, WO2008006481, WO2008006480) and Mochida Pharmaceutical Co., Ltd. (WO2008091021). The principal disadvantage of using said channel agonists as drugs consists in painful affections upon their administration.

The discovery of TRPV1 antagonists primed extensive research both in scientific research institutes and in pharmaceutical companies resulting in the formulation of a therapeutic potential of the compounds. They were recommended for use in the treatment of diseases accompanied with manifestations of chronic pain conditions (RU 2151014, RU 2396261, RU 2448108, RU 2450006, RU 2452733, RU 2458055, RU 2468020, RU 2621708, RU 2621708, EP 2352726, US 2017266139, US 6248788, JP 2016047822) and for the treatment or prevention of diseases or conditions, in which the activation of the TRPV1 ion channel plays a role or is involved, such as migraine (RU 2448108), osteoarthritis (RU 2151014, RU 2448108, RU 2458055, RU 2621708), overactive bladder (RU 2151014, RU 2448108, RU 2450006, RU 2452733, RU 2458055, RU 2468020, RU 2621708, EP 2352726), cough (Khalid S. et al. Transient receptor potential vanilloid 1 (TRPV1) antagonism in patients with refractory chronic cough: a double-blind randomized controlled trial // J Allergy Clin. Immunol. 2014, 134, 56-62; Cabral L.D.M. & Giusti-Paiva A. The Transient Receptor Potential Vanilloid 1 Antagonist Capsazepine Improves the Impaired Lung Mechanics during Endotoxemia // Basic & Clinical Pharmacology & Toxicology, 2016, 119, 421-427; EP 2352726, RU 2448108), inflammatory airway diseases (Lv H. et al. Effect of transient receptor potential vanilloid-1 on cough hypersensitivity induced by particulate matter 2.5 //Life Science, 2016,151,157-166) including asthma (RU 2151014, RU 2448108), inflammatory bowel diseases (IBD) including irritable bowel syndrome (IBS) (RU 2151014, RU 2448108, RU 2452733, RU 2458055, RU 2621708), peptic ulcer (RU 2151014, RU 2448108, RU 2621708), diabetes (Gram D.X. et al. TRPV1: A Potential Therapeutic Target in Type 2 Diabetes and Comorbidities? // Trends in Molecular Medicine, 2017, 23,1002-1013; Zhong, B. et al. TRPV1 mediates glucose-induced insulin secretion through releasing neuropeptides // In Vivo (Brooklyn), 2019, 33, 1431-1437; RU 2448108) and dermatitis (Yun, J.W. et al. TRPV1 antagonist can suppress the atopic dermatitis-like symptoms by accelerating skin barrier recovery // Journal of Dermatological Science, 2011, 62, 8-15; RU 2448108).

In relation to a number of TRPV1 antagonist-based drugs for treating chronic pain, phase I clinical trial data were published: SB-705498 (GSK), AMG-517 (Amgen), MK-2295 (Merck), AZD-1386 (Astra-Zeneca) and GRC-6211 (Lilly/Glenmark), (Gunthorpe MI et al. Clinical development of TRPV1 antagonists: target in pivotal point in the pain pathway // Drug Discov Today, 2009, 14, 56-67; Khairatkar-Joshi N. et al. TRPV1 antagonists: the challenges for therapeutic targeting // Trends Mol. Med. 2009, 15, 14-22). It was shown that a high dose (400 mg) of SB-705498 that has the elimination half-life of 35-93 hours evidences a target pharmacodynamic activity in the reduction of inflammation induced by heat injuries, UV radiation and occurring after the use of capsaicin (Gunthorpe M.J. et al. Clinical development of TRPV1 antagonists: targeting a pivotal point in the pain pathway // Drug Discov. Today 2009,14, 56-67; Chizh B.A. et al. The effects of the TRPV1 antagonist SB-705498 on TRPV1 receptor-mediated activity and inflammatory hyperalgesia in humans // Pain 2007, 132, 132-141). AMG-517 that has the elimination half-life of 13 to 23 days induces a high and steady temperature increase in humans that is reduced only in part with repeated dosing (Gawa N.R. et al. Repeated administration of vanilloid receptor TRPV1 antagonists attenuates hyperthermia elicited by TRPV1 blockade //1 Pharmacol. Exp. Ther. 2007, 323, 128-37). It is determined that ABT-102 relative to AMG-517 demonstrates a slight temperature increase which is quickly attenuated with a repeated dosing of the drug (Honore P. et al. Repeated dosing of ABT-102, a potent and selective TRPV1 antagonist, enhances TRPV1-mediated analgesic activity in rodents, but attenuates antagonist-induced hyperthermia // Pain 2009, 142, 27-35).

The further studies with the use of oral administration of TRPV1 channel antagonists such as SB-705498, AMG-517, MK-2295 and GRC-6211 are postponed or suspended at present. The major impediment to the use of most of said agents in clinic is their unsatisfactory safety profile, notably hyperthermia.

TRPV1 antagonists that have no hyperthermic effect are APHC1, APHC2 and APHC3 - the first peptide inhibitors of the channel which were found in the venom of the sea anemone *Heteractis crispa.* They have an evident analgesic effect when administered intravenously at the doses of 0.01 mg/kg that is related to a partial inhibition of the TRPV1, what was evidenced in different pain stimulation models on experimental animals (RU2368621 C1, 27.09.2009; RU2404245, C1, 20.11.2010; RU2619170, C2, 12.05.2017).

The HCRG21 peptide (accession number P0DL86 in the UniProt database) related to the compounds APHC1, APHC2 and APHC3 that can be produced biotechnologically or isolated from a sea anemone extract is also a TRPV1 channel antagonist (Monastyrnaya M. M. et al. Kunitz-type peptide HCRG21 from the sea anemone Heteractis crispa is a full antagonist of the TRPV1 receptor // Mar. Drugs 2016, 14, 229-234). The potential of the HCRG21 peptide as an analgesic was not disclosed before.

### Summary of the invention

We show in this document the possibility of using the HCRG21 peptide as an analgesic, an agent decreasing the body temperature and/or an anti-inflammatory drug, what is supported by the data obtained in several animal models. The advantage of the HCRG21 peptide over the APHC1 peptide consists in the duration of analgesic effect detected in the course of comparative experiments. The HCRG21 peptide is also characterized by a marked hypothermic and anti-inflammatory effect. So a HCRG21 peptide-based drug outperforms the known analogs.

The invention solves the task of developing an active pharmacological substance with prolonged analgesic action and/or evident hypothermic and anti-inflammatory effect and preparing on the basis of the substance a drug to alleviate painful conditions in a human or animal with parenteral or intranasal administration.

The task is solved by the use of the HCRG21 peptide which has a prolonged analgesic action, a marked hypothermic effect and anti-inflammatory activity. The peptide is an active pharmacological substance introduced within a final dosage form to produce a drug for parenteral or intranasal administration.

The obtained drug is intended to alleviate acute pain conditions and pain during chronic pathologies where the normal functional activity of the TRPV1 ion channel is disturbed and the increased activity is actuated. The drug relieves inflammation, significantly decreases the body temperature of mammals including humans.

Therefore, this invention relates to the HCRG21 peptide for use as a drug with prolonged analgesic, anti-inflammatory and/or hypothermic action.

Preferably the HCRG21 peptide is produced recombinantly.

Specifically, the invention provides for the use of the HCRG21 peptide parenterally or intranasally at the dose of at least 0.1 mg/kg.

The HCRG21 peptide-based drug is intended for treating patients, notably for alleviating pains caused by abdominal operations, joint and cancer pains.

The patient can be a mammal including a human.

In another aspect the invention provides the HCRG21 peptide for use as a drug for treating acute and chronic diseases: migraine, osteoarthritis, cough, overactive bladder, inflammatory airway disease, asthma, inflammatory bowel diseases, irritable bowel syndrome, diabetes, dermatitis.

Preferably the HCRG21 peptide is produced recombinantly.

The invention also proposes a method for treating which comprises administering of a drug comprising the HCRG21 peptide to provide a prolonged analgesic, anti-inflammatory and hypothermic effect.

Specifically, the drug administered according to the method comprises an effective amount of the HCRG21 peptide.

An effective amount of the HCRG21 peptide is the amount sufficient to achieve a specific claimed goal such as the elimination or alleviation of a pain syndrome, the relieve or reduction of inflammation, the decrease of the body temperature. An "effective amount" can be determined experimentally using the known methods related to the claimed goal.

The technical effect of the invention consists in a significantly longer analgesic action of the claimed drug observed in animal pain models (at least 13 hours) with the intravenous, subcutaneous, intramuscular and intranasal administration to mice at the dose of 0.1 mg/kg.

The invention discloses the information on biological properties of the HCRG21 peptide obtained from testing the peptide in different pain sensitivity models on animals. In the comparative tests the claimed peptide outperforms APHC1 - APHC3 analogs in the following parameters:
- a longer analgesic action
- a longer latent period of the first response to pain induced by capsaicin, a reduced licking time and a reduced number of paw licking episodes;
- a more marked hypothermic effect.

The better performance of the claimed peptide in comparison with the closest analogs is determined by fundamental differences in the mechanism of action on a biological target. The APHC1 and APHC3 analogs are polymodal and, depending of the stimulus intensity (agonist (capsaicin) concentration, pH, temperature), they either potentiate the TRPV1, or inhibit (down to 32%) the current going through the channel (Andreev Y. A. et al. Polypeptide modulators of TRPV1 produce analgesia without hyperthermia // Mar. Drugs 2013, 11(12):5100-15), while the HCRG21 peptide completely blocks (inhibition 95%) capsaicin-induced ion current through the cell membrane.

### Brief description of the drawings

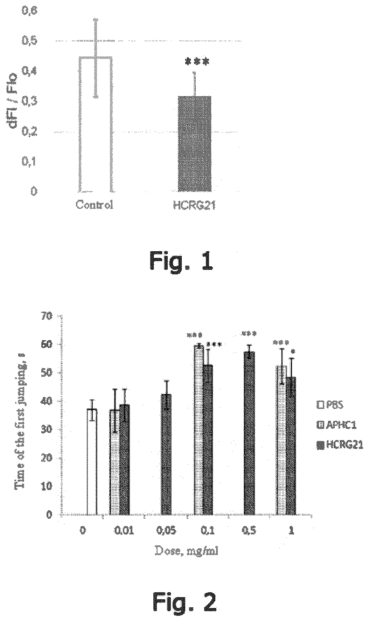

Fig. 1. Inhibition of the intracellular calcium level on the CHO cell line stably expressing the TRPV1 ion channel in response to the addition of 3 µM of capsaicin solution. The peptide HCRG21 concentration of 30 nM is used. Statistical significance of differences is calculated using the Student's t-criterion, the value *** - p<0.001 is considered significant compared to the control.
Fig. 2. Analgesic effect of different HCRG21 peptide doses in a hot plate test by the intramuscular injection. The response time to pain stimulus is plotted on the ordinate. Control animals receive the same volume of physiological saline solution (PBS). For comparison the APHC1 peptide analog is measured at the doses of 0.01, 0.1 and 1 mg/kg. Statistical significance of differences is calculated using the Student's t-criterion, the value ^{∗}- p<0.05, ^{∗∗}- p<0.01, ^{∗∗∗}-p<0.001 is considered significant compared to the PBS group.

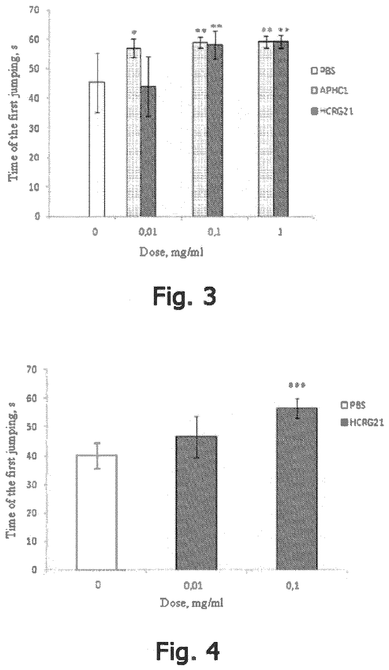

Fig. 3. Analgesic effect of different doses of the HCRG21 peptide and the APHC1 peptide analog in a hot plate test by the intravenous administration. The response time to pain stimulus is plotted on the ordinate. Control animals receive the same volume of PBS solution. Statistical significance of differences is calculated using the Student's t-criterion, the value ^{∗}- p<0.05, ^{∗∗}-p<0.01 is considered significant compared to the PBS group.
Fig. 4. Analgesic effect of different HCRG21 peptide doses in a hot plate test by the intranasal administration. The response time to pain stimulus is plotted on the ordinate. Control animals receive the same volume of PBS solution. Statistical significance of differences is calculated using the Student's t-criterion, the value ^{∗∗∗}- p<0.001 is considered significant compared to the PBS group.

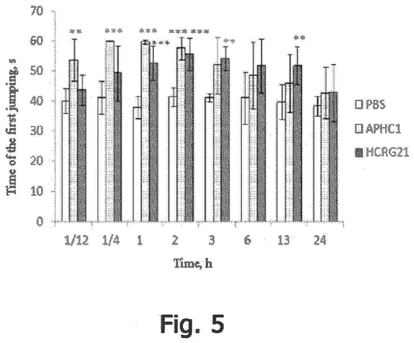

Fig. 5. Pharmacodynamics of analgesic effect of the HCRG21 peptide and the APHC1 peptide analog in a hot plate test at the dose of 0.1 mg/kg by the intramuscular injection. The response time to pain stimulus is plotted on the ordinate and the time between the peptide administration and the measurement of the effect is plotted on the abscissa. Statistical significance of differences is calculated using the Student's t-criterion, the value **- p<0.01, ***- p<0.001 is considered significant compared to the control group of animals administered with the equal PBS volume.

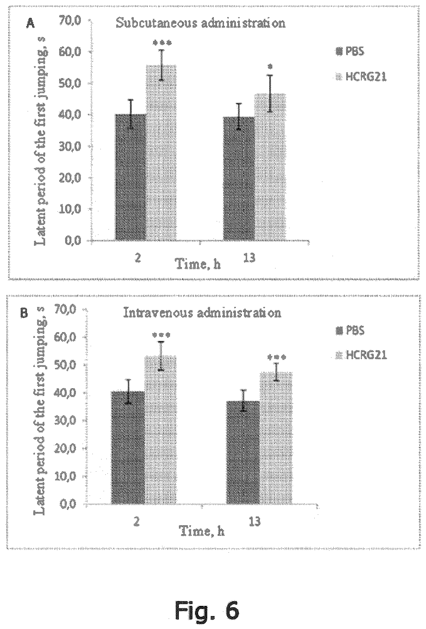

Fig. 6. Analgesic effect of 0.1 mg/kg dose of the HCRG21 peptide in a hot plate test by the subcutaneous (A) and intravenous (B) administration for two time point measurements of the effect at 2 and 13 hours after the drug administration. The response time to pain stimulus is plotted on the ordinate. Control animals receive the same volume of PBS solution. Statistical significance of differences is calculated using the Student's t-criterion, the value ^{∗}- p<0.05, ^{∗∗∗}- p<0.001 is considered significant compared to the PBS group.

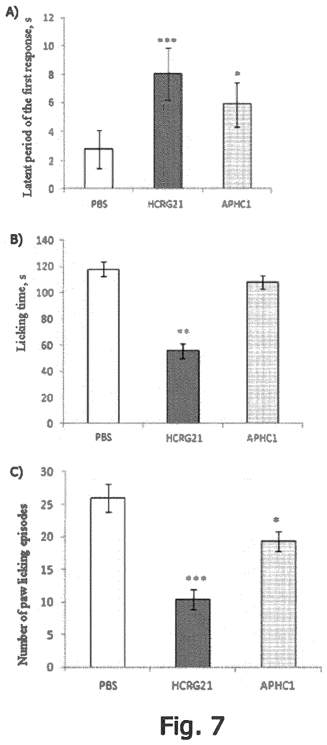

Fig. 7. Analgesic effect of the HCRG21 peptide and the APHC1 peptide analog at the dose of 0.1 mg/kg by the intramuscular injection in a capsaicin test. The measured parameters recorded during 15 minutes of observation are plotted on the ordinate: (A) latent period of the first response, (B) paw licking time, (C) number of paw licking episodes. Control animals receive the same volume of PBS solution. Statistical significance of differences is calculated using the Student's t-criterion, the value *- p<0.05, **- p<0.01, ***- p<0.001 is considered significant compared to the PBS group.

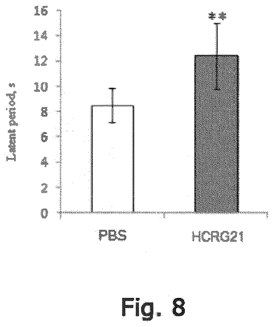

Fig. 8. Anti-inflammatory effect of HCRG21 peptide at the dose of 0.1 mg/kg by the intramuscular injection in a test of thermal hypersensitivity induced by the intraplantar administration of Complete Freund's Adjuvant. The time of inflamed paw withdrawal from the hot plate (t=53°C) is plotted on the ordinate. Statistical significance of differences is calculated using the Student's t-criterion, the value **- p<0.01 is considered significant compared to the control group of animals administered with the equal PBS volume.

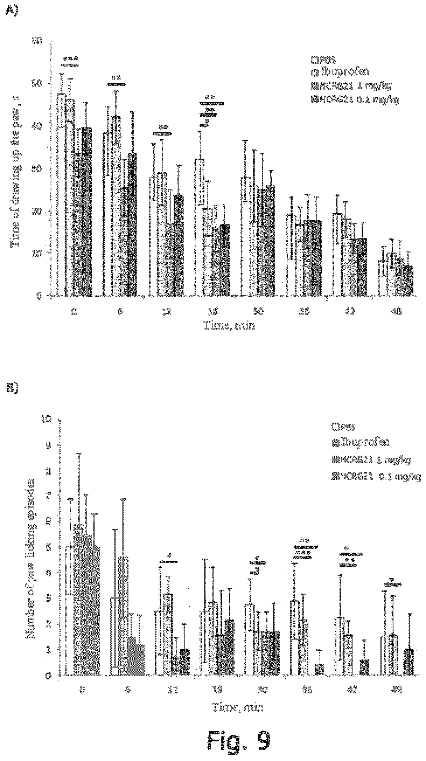

Fig. 9. Analgesic effect of the HCRG21 peptide at the doses of 0.1 and 1 mg/kg by the intramuscular injection in a formalin test. Control animals receive the same volume of PBS solution (negative control) or ibuprofen solution at the dose of 100 mg/kg (positive control). The measured parameters recorded during 1 minute of observation are plotted on the ordinate: (A) a latency time of hind paw withdrawal, (B) a number of licking episodes of hind paw, the time between the peptide administration and the start of the next measurement is plotted on the abscissa. Statistical significance of differences is calculated using the Student's t-criterion, the value ^{∗}-p<0.05, ^{∗∗}- p<0.01, ^{∗∗∗}- p<0.001 is considered significant compared to the PBS group.

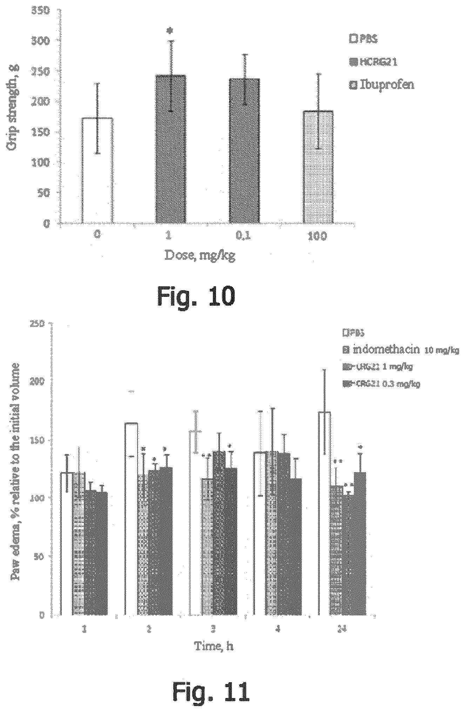

Fig. 10. Analgesic effect of the HCRG21 peptide at the doses of 0.1 and 1 mg/kg by the intramuscular injection in a mechanical hypersensitivity test under conditions of acute local inflammation induced by intraplantar administration of carrageenan. Control animals receive the same volume of PBS solution (negative control) or ibuprofen solution at the dose of 100 mg/kg (positive control). Grip strength is plotted on the ordinate. Statistical significance is calculated using the Student's t-criterion, the value *- p<0.05 is considered significant compared to the PBS group.
Fig. 11. Anti-inflammatory effect of the HCRG21 peptide at the doses 0.1 and 1 mg/kg by the intravenous administration in a test of acute local inflammation induced by intraplantar administration of carrageenan. Control animals receive the same volume of PBS solution (negative control) or indomethacin solution at the dose of 10 mg/kg (positive control). The increase of the paw volume in percent relative to the initial volume is plotted on the ordinate. Statistical significance of differences is calculated using the Student's t-criterion, the value ^{∗}- p<0.05, ^{∗∗}-p<0.01 is considered significant compared to the PBS group.

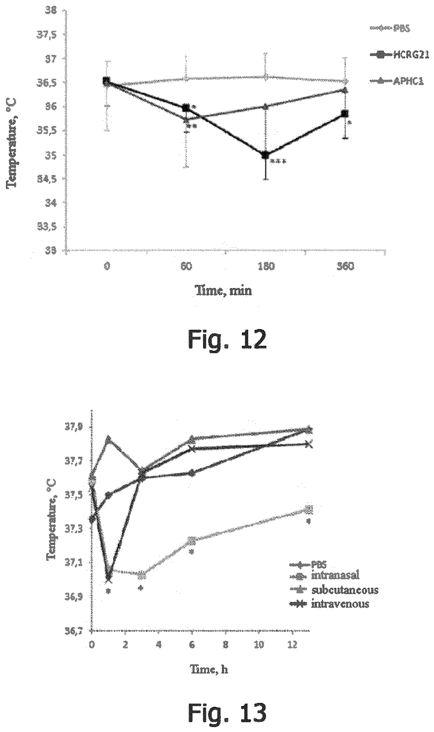

Fig. 12. Effect of the HCRG21 peptide and the APHC1 peptide analog on the rectal body temperature of ICR cell mice by the intramuscular injection at the dose 0.1 mg/kg. Statistical significance of differences is calculated using the Student's t-criterion, the value ^{∗}- p<0.05, ^{∗∗}-p<0.01, ^{∗∗∗}- p<0.001 is considered significant compared to the control group of animals administered with the equal PBS volume.
Fig. 13. Effect of the HCRG21 peptide on the rectal body temperature of ICR cell mice by different methods of administration at the dose 0.1 mg/kg. Statistical significance of differences is calculated using the Student's t-criterion, the value * - p<0.05 is considered significant compared to the control group of animals administered with the equal PBS volume.

### Detailed description of embodiments

### Example 1

### Production of the HCRG21 peptide

The active peptide is produced by a biotechnological method as a fused protein with a thioredoxin domen. The synthesis of the hcrg21 gene was carried out by PCR (2 steps) using gene specific primers created on the basis of the nucleotide sequence of the hcrg21 gene optimized according to rare codons for *E. coli* expression completely overlapping the nucleotide sequence (Monastyrnaya M.M. et al. Kunitz-type peptide HCRG21 from the sea anemone Heteractis crispa is a full antagonist of the TRPV1 receptor // Mar. Drugs 2016, 14, 229-234). The restriction sites for EcoRI and Xhol are introduced respectively into the sequences of forward and reverse flanking primers. An additional ATG codon encoding the methionine residue is introduced in the sequence of the forward flanking primer upstream the sequence encoding the first amino acid residue of HCRG21 peptide. A TAA stop codon is added in the reverse primer sequence downstream the Xhol restriction site. A genetically engineered construct (expression vector) capable of expressing the HCRG21 peptide in *E. coli* cells is produced on the synthesized hcrg21 gene fragment by the ligation with the pET32b+ expression vector (Novagen) after the hcrg21 gene fragment and the pET32b+ plasmid are treated with EcoRI and Xhol restrictases. The obtained plasmids are checked by sequencing. This results in the production of a plasmid bearing TrxA-HCRG21 fused protein genes under T7 promoter control.

The fused protein for the HCRG21 peptide synthesis can comprise any other commercially available helper protein (e.g. barnase (bar'), disulfide exchange protein (DsbC), maltose binding protein (MBP), or a combination of chitin binding domain and *Synechocystis sp.-* derived miniinteine DnaB capable of undergoing pH-dependent self-cleavage). The TrxA-HCRG21 fused protein is a fused amino acid sequence of a thioredoxin domen A(TrxA, 109 amino acid residues), a sequence of 6 histidine residues, a methionine residue located just upstream the first HCRG21 peptide residue and the target HCRG21 peptide.

BL21(DE3) cells are transformed by an expression vector comprising a sequence encoding the fused protein. After being selected on plate with selective antibiotic (carbenicillin), the cells are transferred to a flask with 1200 ml of liquid-(LB) media with the selective antibiotic (carbenicillin) and are cultured under stirring up to optical density ~ 0.6 units (A₆₀₀) at the temperature of 37°C. The expression is induced by the addition of isopropyl-β-D-1-thiogalactopyranoside to the concentration of 0.4 mM. The cells are further incubated under stirring at 19°C to produce a fused protein and after 20 hours are precipitated and frozen. For disintegration the cells are resuspended in a buffer solution (20 mM tris-HCl pH 7.2, 150 mM NaCl). The cells are disintegrated by ultrasound, the cell debris is precipitated by centrifugation. The target fused protein is isolated from the soluble fraction.

The product is separated from the other cell proteins with the use of metal-affinity chromatography on a Ni²⁺-sorbent in a buffer solution (20 mM Tris-HCl pH 7.2, 150 mM NaCl), a buffer solution (150 mM imidazole, 300 mM NaCl, 20 mM Tris, pH 7.5) is used for eluting the fused protein. Then the affinity tag and the helper protein are removed by cleaving the fused protein with cyan bromide at the methionine residue added intentionally upstream the first amino acid of the HCRG21 peptide. The fused protein is dissolved in 0.1 M HCl to the protein concentration of 1 mg/ml, cleaved with cyan bromide for 18 hours in the dark using a 600-fold molar excess of cyan bromide. The solvent and the cyan bromide excess are removed in a vacuum concentrator, further the hydrolysis product mixture is injected onto a column with reverse phase sorbent Jupiter C18 (10x250 mm, Phenomenex, Torrance, CA, USA). The fractionation do in a linear gradient of acetonitrile concentration in 0.1% (v/v) trifluoroacetic acid. Mass spectrometric analysis is used to control the purity of the prepared recombinant peptide.

### Example 2

### Verification of a correct folding of the recombinant analog

Testing is carried *in vitro* on a CHO cell culture stably expressing the TRPV1 ion channel. The stable cell line is unfrozen from the cryobank and is cultured for at least 2 weeks on a medium (DMEM /10% fetal calf serum / 1% PS (penicillin /streptomycin)) added with B/Z antibiotic mixture (Blasticidin S (5 µg /ml), zeocin (250 µg /ml)) in sterile flattened bottles at 37°C in the presence of 5% CO₂ with splitting at regular intervals every 2-3 days. For measuring, the cells are placed in 96-well plates with black walls and transparent bottom in the amount of 75000 cells per well and are cultured for a night at 37°C in the presence of 5% CO₂ in a complete medium without PS antibiotics containing 1 µg/ml of tetracycline.

The cells are stained with cytoplasmic calcium dye Fluo-4AM using the methods the and the ready to use kit Fluo-4 Direct^{™} (Invitrogene) for calcium assay and are sequentially incubated in the dark at 37°C for 45-60 minutes and then at 25°C for 45-60 minutes. Measurements take place at a room temperature with the use of a plate spectrophotometer equipped with a built-in automated liquid injector system NOVOstar (BMG LABTECH, Germany). Selective ion currents through the TRPV1 ion channel (enhancement of the dye due to the increased concentration of intracellular calcium) is induced by the addition of 3 µM of capsaicin solution with a dosing device in each well during the measurement. Fluorescent signals are recorded from the bottom at the following parameters (λex = 485 nM, λem = 520 nM, measuring interval: 10 flashes every 2 s). CHO cells without the TRPV1 ion channel are used as a negative control, the maximal fluorescence is measured 20 seconds after the addition of capsaicin. The tested HCRG21 peptide is placed in a well before the measurement so that the final concentration thereof is 30 nM, the control wells are filed with an equal volume of a buffer solution. The HCRG21 peptide induces a reliable decrease in the intracellular calcium level (Fig. 1) calculated as a difference between the maximal and the basic fluorescence (dFl) divided by the basic fluorescence (Flₒ). The results are averaged by 9 independent measurements, statistical significance of differences between the control and experimental group is determined using the Student's t-criterion.

### Example 3

### Testing of analgesic effect of the HCRG21 peptide on mice in a hot plate test by different methods of administration

Mice are divided into 19 groups (3 control and 16 experimental ones) of 9 mice each. Tests are performed on ICR male white mice of 20-30 g body weight. Pain stimulation is done by placing a mouse on a metal surface of instrument heated to 55°C. For preventing paw burns, the time of exposure is restricted with 60 seconds. The tested sample of the HCRG21 peptide is dissolved in a sterile physiological saline solution and is administered at the dose of 0.01, 0.05, 0.1, 0.5 and 1 mg/kg. For determining the most effective method of administration different ways of administration are used: intramuscular, intravenous, intranasal. The APHC1 peptide is used as control to compare the effectiveness of analgesic action under the same conditions with the intramuscular and intravenous administration. Control animals receive the equivalent volume of sterile physiological saline solution. The analgesic effect is measured by the increase of time from the moment of placing an animal on the plate to the first jumping. The pain sensitivity is measured 60 min after the intramuscular (Fig. 2), 30 min after the intravenous (Fig. 3) and 120 min after the intranasal administration (Fig. 4) of the drugs. According to the obtained results it is determined that with the intramuscular injection the analgesic effect of the HCRG21 peptide occurs at the dose of 0.1 mg/kg, the APHC1 having a little higher effect consistent with the effect of HCRG21 at the dose of 0.5 mg/kg. The increase of the dose up to 1 mg/kg results in a slight decrease of the effectiveness of both peptides (Fig. 2). With the intravenous administration the control sample is effective in the dose range of 0.01 to 1 mg/kg, while the HCRG21 is effective in the range of 0.1 to 1 mg/kg (Fig. 3). With the intranasal administration the HCRG21 peptide activity is maintained at the same minimal dose of 0.1 mg/kg. The results are statistically processed, statistical significance of differences of the results of control and experimental group is determined using the Student's test. It is determined that at the dose of 0.1 mg/kg the HCRG21 peptide efficiently reduces the pain sensitivity by any administration methods.

### Example 4

### Testing of the duration of analgesic effect of the HCRG21 peptide on mice in a hot plate test by the intramuscular injection

Mice are divided into 24 groups (control and experimental ones) of 9 mice each. Tests are performed on ICR male white mice of 20-30 g body weight. Pain stimulation is simulated by placing a mouse on a metal surface of instrument heated to 55°C. For preventing paw burns, the time of exposure is restricted with 60 seconds. The tested sample of the HCRG21 peptide is dissolved in a sterile physiological saline solution and is administered intramuscularly at the dose of 0.1 mg/kg. The APHC1 peptide is used as control to compare the duration of analgesic action at the same concentration under the same conditions. Control animals receive the equivalent volume of sterile physiological saline solution. The analgesic effect is measured by the increase of time from the moment of placing an animal on the plate to the first jumping (Fig. 5). The pain sensitivity is measured 5 min, 15 min, 1 hour, 2, 3, 6, 13 and 24 hours after the administration of the drug. According to the obtained results it is determined that the analgesic action of HCRG21 is developed gradually, is significantly different from the control by 1 h (the maximal effect is 2 h) and is prolonged (at least 13 h). This distinguishes the action of the HCRG21 peptide from the APHC1 analog which quickly provides an analgesic effect but only for 2 hours. Each mouse is used only for one measurement of the analgesic effect. The results are statistically processed, statistical significance of differences of the results of control and experimental group is determined using the Student's test.

### Example 5

### Testing of the duration of analgesic effect of the HCRG21 peptide on mice in a hot plate test by different methods of administration

Mice are divided into 8 groups (4 control and 4 experimental ones) of 7 mice each. Tests are performed on ICR male white mice of 20-30 g body weight. Pain stimulation is simulated by placing a mouse on a metal surface of instrument heated to 55°C. For preventing paw burns, the time of exposure is restricted with 60 seconds.

The tested sample of the HCRG21 peptide is dissolved in a sterile physiological saline solution and is administered at the dose of 0.1 mg/kg intravenously and subcutaneously. Control animals receive the equivalent volume of sterile physiological saline solution. The analgesic effect is measured by the increase of time from the moment of placing an animal on the plate to the first jumping (Fig. 6). The pain sensitivity is measured 2 and 13 h after the administration of the drug.

According to the obtained results it is determined that the analgesic action of HCRG21 is significantly different from the control and is prolonged (at least 13 h) both in the case of a subcutaneous (Fig. 6A) and intravenous administration (Fig. 6B). Each mouse is used only for one measurement of the analgesic effect. The results are statistically processed, statistical significance of differences of the results of control and experimental group is determined using the Student's test.

### Example 6

### Testing of analgesic effect of the HCRG21 peptide in a capsaicin test

Tests are performed on ICR male white mice of 20-30 g body weight. Mice are divided into 3 groups (a control one and experimental ones) of 9 mice each. The tested HCRG21 peptide is dissolved in a sterile physiological saline solution and is administered intramuscularly at the dose of 0.1 mg/kg. The APHC1 peptide is used as control to compare the effect at the same concentration under the same conditions. Control animals receive the equivalent volume of sterile physiological saline solution. 60 minutes later all mice are injected in a hinder paw pad with 20 µl of the mixture (at the ratio of 1:9 (v/v)) of 10% alcoholic capsaicin solution with physiological saline solution. The amount of capsaicin in 20 µl of the mixture solution is 3 µg. The observance of the mouse behavior starts directly after the capsaicin injection and the first response of the animal to pain induction is recorded (the first licking or twitching of the injured paw), then a number of licking episodes and the time of licking of the injured paw are recorded. Each animal is observed for 15 min. The results are statistically processed, statistical significance of differences of the results of control and experimental group is determined using the Student's test.

The analgesic effect is measured by the increase of time from the moment of placing an animal on the plate to the onset of the response of the animal to pain induction (Fig. 7A) and by the decrease of the duration of licking (Fig. 7B) and a number of injured paw licking episodes (Fig. 7C). According to the obtained results it is determined that the analgesic effect of the HCRG21 is expressed in a reduced sensitivity of animals to capsaicin irritating action: the intensity of licking and the time of licking of the irritated paw is decreased more than 2.5 times and the time of onset of the behavioral response of an animal to pain induction is increased in 3.5 times in comparison with the control group. The analgesic effect of the comparative peptide APHC1 is less pronounced in all three recorded parameters.

### Example 7

### Testing of anti-inflammatory effect of the HCRG21 peptide in a thermal hypersensitivity model

Tests are performed on ICR male white mice of 20-30 g body weight. Mice are divided into 2 groups (a control and an experimental one) of 9 mice each. Mice of the experimental group are injected in a hinder paw pad with an inflammatory agent represented by 20 µl of a mixture of Complete Freund's Adjuvant and physiological saline solution at the ratio of 1:1 (v/v). 24 hours later the animals receive an intramuscular injection of 200 µl of the tested HCRG21 peptide solution in sterile physiological saline solution at the dose of 0.1 mg/kg; the animals of the control group receive 200 µl of physiological saline solution. The measurement takes place 60 minutes after the administration. A latent time of withdrawal of the paw exposed to the inflammatory agent from the hot plate (t = 53°C) is recorded. The analgesic effect is measured by the increase of time from the moment of placing an animal on the plate to the first licking of the hind paw (Fig. 8). The results are statistically processed, statistical significance of differences of the results of control and experimental group is determined using the Student's test.

### Example 8

### Testing of analgesic effect of the HCRG21 peptide in a formalin test

Tests are performed on ICR male white mice of 20-30 g body weight. Mice are divided into 4 groups (a control one and experimental ones) of 9 mice each. The tested HCRG21 peptide is dissolved in a sterile physiological saline solution and is administered intramuscularly at the doses of 0.1 and 1.0 mg/kg. Control animals receive the equivalent volume of sterile physiological saline solution. Ibuprofen at the dose of 100 mg/kg is used as a positive control and is administered to animals orally. 60 minutes later all mice are injected in a hinder paw pad with 20 µl of 2.5% formalin solution. Patterns of pain response are recorded immediately after the injection (a number of paw licking episodes and a hind paw withdrawal time) according to the following schedule:
1^{st} period (acute): for 20 min after the injection patterns are recorded per 1 min with 6-minute intervals (0, 6, 12 and 18 min);
2^{nd} period (tonic): 30 min after the formalin injection patterns are recorded for 20 min per 1 min with 6-minute intervals (30, 36, 42, 48 min).

The analgesic effect is evaluated by the decrease of the duration of pain response patterns relative to control animals (Fig. 9). The results are statistically processed, statistical significance of differences of the results of control and experimental group is determined using the Student's test.

According to the obtained results it is determined that the analgesic effect of the HCRG21 peptide consists in a significant decrease of a number of paw licking episodes and the time of drawing up the hind paw both in phase I of the acute period of the formalin test (0-10 min) characterizing the interaction with nociceptors and in phase II (10-15 min later) by blocking a pH-mediated or modulated TRPV1 activation, which takes place during the inflammation. Ibuprofen (a typical analgesic of the NSAID group) concedes much to the HCRG21 peptide in its analgesic properties.

### Example 9

### Testing of analgesic effect of the HCRG21 peptide in a model of pain caused by a carrageenan-induced inflammation

Tests are performed on ICR male white mice of 20-30 g body weight. Mice are divided into 4 groups (a control one and experimental ones) of 9 mice each. The tested HCRG21 peptide is dissolved in a sterile physiological saline solution and is administered intramuscularly at the doses of 0.1 and 1.0 mg/kg. Control animals receive the equivalent volume of sterile physiological saline solution. Ibuprofen at the dose of 100 mg/kg is used as a positive control and is administered to animals orally. 60 minutes later all mice are injected in a hind paw pad with 20 µl of 1% carrageenan solution. Immediately after the injection a forceps grip strength is measured (values exceeding 300 are considered as 300). The analgesic effect is evaluated by the increase of grip strength relative to control animals (Fig. 10). The results are statistically processed, statistical significance of differences of the results of control and experimental group is determined using the Student's test. Statistically significant analgesic effect of the HCRG21 is manifested at the dose of 1 mg/kg and is expressed in the increase of the grip strength, what markedly exceeds the indices (dose and grip strength) recorded for ibuprofen.

### Example 10

### Testing of anti-inflammatory effect of the HCRG21 peptide in a carrageenan-induced acute local inflammation model

Tests are performed on ICR male white mice of 20-30 g body weight. Mice are divided into 4 groups (a control and experimental ones) of 6 mice each. The tested HCRG21 peptide is dissolved in a sterile physiological saline solution and is administered intravenously at the doses of 0.1 and 1.0 mg/kg. Control animals receive the equivalent volume of sterile physiological saline solution. Indomethacin at the dose of 10 mg/kg is used as a positive control and is administered to animals orally. The volume of right hind paw is measured in all animals using a pletismometer (Ugo Basile). 30 minutes later mice exposed to the intravenous administration of physiological saline solution or the HCRG21 peptide and 60 minutes later mice receiving orally indomethacin are injected with 20 µl of 1% carrageenan solution (Sigma, CIIIA) in a pad of right hind paw. Then the formed edema is measured using a pletismometer after 1, 2, 3, 4 and 24 hours. The results are statistically processed, statistical significance of differences of the results of control and experimental group is determined using the Student's test. Statistically significant anti-inflammatory effect of the HCRG21 is manifested at the doses of 0.1 and 1 mg/kg and is expressed in a lower edema volume relative to the control and comparable to that in the case of using indomethacin, the anti-inflammatory drug of the NSAID group, at the dose of 10 mg/kg (Fig. 11).

### Example 11

### Effect of the HCRG21 peptide on the body temperature

Tests are performed on ICR male white mice of 20-30 g body weight. Mice are divided into 3 groups (a control one and experimental ones) of 9 mice each. The tested sample of the HCRG21 peptide is dissolved in a physiological saline solution and is administered at the dose of 0.1 mg/kg intramuscularly. The APHC1 peptide is used as control to compare the effect at the same concentration under the same conditions. Control animals receive the equivalent volume of sterile physiological saline solution. Using electronic thermometer inserted rectally the initial body temperature is measured (0 min), then measurements are performed 60, 180 and 360 min after the administration of the peptides (Fig. 12). The results are statistically processed, statistical significance of differences of the results of control and experimental groups is determined using the Student's test.

Both peptides induce statistically significant decrease of the body temperature of the animals by 0.7-1.0°C on the 60^{th} minute after the administration, therewith the HCRG21 has a more prolonged hypothermic effect observed even after 360 min with the maximal decrease of the temperature by 1.5° 180 min after the injection.

### Example 12

### Effect of the HCRG21 peptide on the body temperature by different methods of administration

Tests are performed on ICR male white mice of 20-30 g body weight. Mice are divided into 4 groups (a control and experimental ones) of 7 mice each. The tested sample of the HCRG21 peptide is solved in a sterile physiological saline solution and is administered at the dose of 0.1 mg/kg intranasally, subcutaneously and intravenously. Control animals receive the equivalent volume of sterile physiological saline solution. Using electronic thermometer inserted rectally the initial body temperature is measured (0 min), then measurements are performed 1, 3, 6 and 13 hours after the administration of the peptide (Fig. 13). The results are statistically processed, statistical significance of differences of the results of control and experimental group is determined using the Student's test.

The HCRG21 peptide induces statistically significant decrease of the body temperature of the animals by 0.4-0.5°C on the 60^{th} minute after the intravenous and intranasal administration, therewith the HCRG21 has a more prolonged hypothermic effect observed even after 13 hours after the intranasal administration.

### Example 13

### Testing of locomotor activity in an "open field" test

For analyzing a possible neurotoxic or irritant action of the tested HCRG21 peptide, its effect on the locomotor activity and behavior of animals is evaluated in an "open field" test using the automated actometer OPTO-VARIMEX ATM3 AUTO SYSTEM. Tests are performed on ICR male white mice of 20-30 g body weight. Mice are divided into 7 groups (a control one and experimental ones) of 8 mice each. The tested sample is dissolved in a sterile physiological saline solution and is administered at the doses of 0.01-1.0 mg/kg intramuscularly. The APHC1 peptide is used as control to compare the effect at the same concentration under the same conditions. Control animals receive the equivalent volume of sterile physiological saline solution. 55 min after the administration of the substances an animal is placed on the testing platform of the instrument. The period of stay of an animal on the field is 3 min. Motor activity indices are recorded: Walking distance (DT - Ambulatory distance traveled by the subject), time period when the animal did not move (RT - Resting time), Walking time (AT - Ambulatory time, when the subject), number of vertical episodes (V1C/V1B) rearing.

Data for each group are recorded in a table, the results are presented as an average value ± SEM. Significant differences are presented relative to the control group (1% PBS), ^{∗}- p < 0.05, ^{∗∗} - p < 0.01.

**Table**

| Group | DT, cm | RT, s | AT, s | V1C/V1B |
|---|---|---|---|---|
| PBS | 779.4±14 | 69.3±2.9 | 110.7±2.9 | 10.7±2.5 |
| APHC1 -1 mg/kg | 851 ±17.2 | 70.6±4.3 | 109.4±4.2 | 10.2±2.6 |
| Change relative to control, % | 9.2 | 1.9 | -1.2 | -4.3 |
| APHC1 - 0.1 mg/kg | 1261.4±19^{∗∗} | 55.4±3.8^{∗} | 124.6±3.7^{∗} | 19.9±3.1^{∗} |
| **Change relative to control, %** | 61.8 | -20.0 | 12.6 | 85.5 |
| APHC1 - 0.01 mg/kg | 892.9±13 | 68.4±3.9 | 104.1±3.6 | 19.5±2.4^{∗} |
| Change relative to control, % | 14.5 | -1.3 | -5.9 | 82.0 |
| HCRG21 - 1 mg/kg | 771.5±18.3 | 88.6±5^{∗} | 91.4±5^{∗} | 16.2±1.8^{∗} |
| Change relative to control, % | -1.0 | 27.9 | -17.5 | 51.7 |
| HCRG21 - 0.1 mg/kg | 1399.4±26.9^{∗} | 56.1 ±5.1 | 123.9±5.1 | 21.5±3.3^{∗} |
| Change relative to control, % | 79.5 | -19.0 | 11.9 | 100.7 |
| HCRG21 - 0.01 mg/kg | 863±17.3 | 78.6±4.2 | 101.4±4.2 | 15.4±2.5 |
| Change relative to control, % | 10.7 | 13.5 | -8.4 | 43.5 |

The statistical analysis with the use of the Student's criterion shows that in comparison with the control group in animals of experimental groups in the analyzed parameters no dose-dependent significant differences are recorded. The tested HCRG21 peptide and the control APHC1 have no marked depressing effect on the CNS, on the contrary, their administration at the dose of 0.1 mg/kg results in a weak enhancement of locomotor activity. As both peptides in the tested doses have no neurotoxic side effect on the organism of animals, all specific analgesic effects are considered to be direct.

### Industrial applicability

The above examples evidence that the HCRG21 peptide manifests the analgesic activity on mammals by different administration methods (intramuscular, intravenous, subcutaneous and intranasal).

The effect of the HCRG21 peptide was studied in five different tests on mice of wild type, what supports the possibility of using the peptide as an analgesic to manage pain syndromes of various etiology. The hot plate test is the most related to the inhibition of the TRPV1 ion channel, since the TRPV1 plays an essential role in the sensation of temperature. As capsaicin is a selective agonist of the TRPV1 channel, the efficacy of the peptide is tested as that of an antagonist of the receptor in a capsaicin-induced pain model. A capsaicin injection simulates pain from different pro-inflammatory endogenous and exogenous ligands, what is manifested in licking and shaking of the injected paw. The formalin test is a complex *in vivo* pain model, in which not only an analgesic effect is evaluated but anti-inflammatory action in general. Formalin injections induce in mice a two-phase behavioral response. The first phase (acute) lasts for the first 10 minutes after the administration of formalin and is determined by a direct stimulation of nociceptors; the second phase (chronic) is recorded 10-15 minutes after the formalin injection and lasts for 20-40 minutes, it is related to mechanisms of the progression of neurogenic inflammation and the CNS sensitization. Thermal hyperalgesia induced by the administration of Complete Freund's Adjuvant (CFA) is another variant of an inflammatory model in which different ways of inflammation are involved. The administration of carrageenan induces the development of an inflammatory response and as a consequence a pain resulting from the activation of such nociceptors as TRPA1 and TRPV1 by the inflammation mediators and the change (reduction) of the acidity.

The control peptide APHC1 is used in many experiments for comparison. The HCRG21 demonstrates a more prolonged analgesic effect (at least 13 h) than the APHC1 (less than 3 h) with the intramuscular injection at the dose of 0.1 mg/kg (Fig. 5). The HCRG21 has a longer (8 s) latent period of the first response to capsaicin-induced pain, a reduced time (59 s) and reduced number of licking episodes of the respective paw (10) in comparison with the APHC1 (5, 9, 110 and 18 respectively) with the intramuscular injection at the dose of 0.1 mg/kg (Fig. 7). The HCRG21 in comparison with the APHC1 has a longer hypothermic effect which is observed even 360 min after the administration (Fig. 12).

The claimed agent exhibits a significant analgesic effect with the intramuscular injection in a formalin test at the doses of 0.1-1.0 mg/kg exceeding the effect of the analgesic and anti-inflammatory drug widely adopted in practice (Fig. 9).

The HCRG21 in the open field test has no negative effect on the CNS (Table) and its analgesic and anti-inflammatory action relates to the effect of specific inhibition of the TRPV1 channel.

The foregoing allows using the HCRG21 peptide individually or as an active ingredient of a final analgesic dosage form for veterinary and medicine purposes. Drugs containing the claimed agent as an active form can be administered parenterally or intranasally.

All publications or patents cited therein are included in this document by reference in their entirety, for they show the state of the art at the time of this invention and/or provide the description and the implementation of the present invention.

## Claims

1. HCRG21 peptide for use as a drug with prolonged analgesic, anti-inflammatory and/or hypothermic effect.

2. The HCRG21 peptide according to claim 1, wherein the HCRG21 peptide is produced recombinantly.

3. The HCRG21 peptide according to claim 2, wherein the HCRG21 peptide is used parenterally or intranasally at the dose of at least 0.1 mg/kg.

4. The HCRG21 peptide according to claim 2, wherein the HCRG21 peptide is used in mammals, specifically in a human.

5. The HCRG21 peptide according to any one of claims 2 to 4, wherein the HCRG21 peptide is used for alleviating pains caused by abdominal operations, joint and cancer pains.

6. The HCRG21 peptide for use as a drug for treating acute and chronic diseases such as migraine, osteoarthritis, cough, overactive bladder, inflammatory airway disease, asthma, inflammatory bowel diseases, irritable bowel syndrome, diabetes, dermatitis.

7. The HCRG21 peptide according to claim 6, wherein the HCRG21 peptide is produced recombinantly.
